# EUROPÄISCHE PATENTSCHRIFT

(11) **EP 1 503 706 B1**
(45) Veröffentlichungstag und Bekanntmachung des Hinweises auf die Patenterteilung: **23.04.2008**
(21) Anmeldenummer: 03714789.9
(22) Anmeldetag: 07.03.2003
(51) Int. Cl.: A61F 2/52, A61F 2/50, B29C 43/20

(54) **BRUSTPROTHESE**
BREAST PROSTHESIS
PROTHESE DU SEIN

(30) Priorität: 08.05.2002 DE 10220594
(43) Veröffentlichungstag der Anmeldung: 09.02.2005
(73) Patentinhaber: Dekumed Gesellschaft für Kunststoff- und Medizintechnik Gmbh & Co. Kg, D-83233 Bernau (DE)
(72) Erfinder: KRAMER, Bernd, 83250 Marquartstein (DE); RICKAUER, Peter, 83233 Bernau (DE)
(74) Vertreter: Westendorp, Michael Oliver
(86) Internationale Anmeldenummer: PCT/EP2003/002355
(87) Internationale Veröffentlichungsnummer: WO 2003/094778

(56) Entgegenhaltungen:
- DE-A- 3 336 279
- DE-A- 3 942 608
- DE-A- 19 754 144
- DE-C- 3 938 328
- DE-C- 4 421 516
- DE-U- 29 708 120
- FR-A- 2 432 301
- US-A- 5 340 352
- US-A- 6 113 634

## Beschreibung

Die Erfindung betrifft eine Brustprothese mit einem schalenförmigen vorderen Teil, enthaltend einen Weichkunststoff und einem dem Körper zugewandten rückwärtigen Teil mit einem Weichkunststoff sowie einem dazwischenliegenden Einsatzkörper.

Brustprothesen aus einer gelartig ausgehärteten Kunstharzmasse, die zwischen zwei miteinander verbundenen thermoplastischen Folien eingeschlossen sind, sowie ein Verfahren zur deren Herstellung sind beispielsweise aus der DE-C-33 36 279 bekannt. In dieser Patentschrift ist auch eine Form beschrieben, mit deren Hilfe die Brustprothese hergestellt werden kann. Die Brustprothese enthält einen Vollkörper aus einer Silikon-Kunststoffmasse.

Ein Verfahren zur Herstellung einer Brustprothese ist ferner aus der DE-C-39 38 328 bekannt. Diese Brustprothese besteht im wesentlichen aus einer gelartig härtbaren Kunstharzmasse, die zwischen zwei thermoplastischen Folien eingeschlossen ist. Ferner ist in dieser Patentschrift eine Vorrichtung zur Herstellung einer derartigen Prothese angegeben.

In beiden Fällen handelt es sich um Vollprothesen, die ein verhältnismäßig hohes Gewicht haben.

Aus der DE-C-44 21 516 ist eine Brustprothese für brustamputierte Frauen und ein Verfahren zu deren Herstellung bekannt. Die Brustprothese weist einen weich-elastischen Kunststoffkörper auf, der aus zwei längs ihres gemeinsamen Randes verbundenen Kunststofffolien und einer zwischen dem Kunststofffolienhohlraum frei eingeschlossenen Kunststoffmasse besteht. Die der Trägerin zuwendbare Rückseite des Kunststoffkörpers ist mit einem Stoffteil bedeckt, das an dem Kunststoffkörper befestigt ist. Der Stoffteil besteht aus einem thermoplastischen Material und ist mit dem Rand der Kunststofffolien verschweißt. Nach einer speziellen Ausführungsform enthält die Rückseite des Kunststoffkörpers eine Vertiefung, in der ein Polsterkörper angeordnet ist; und das Stoffteil hat eine schlitzförmige Öffnung, durch die der Polsterkörper in die Vertiefung einführbar und daraus entnehmbar ist. Der Polsterkörper kann beispielsweise aus einem Schaumkunststoff oder einem Faserknäuel bestehen. Durch den Schaumkunststoff wird zwar in erwünschter Weise das Gewicht der Prothese vermindert; der Polsterkörper kann sich aber innerhalb des Hohlraumes verschieben. Außerdem nimmt der Polsterkörper durch die schlitzförmige Öffnung Feuchtigkeit auf, wodurch die Prothese wieder schwerer und eine Reinigung des Polsterkörpers notwendig wird.

Aus der DE-A-197 54 144 ist ein Verfahren zur Herstellung einer Brustprothese mit einem etwa schalenförmigen Prothesenkörper bekannt, der gebildet wird durch Aufeinanderlegen von wenigstens zwei Kunststoffolien, die durch Randverbindung zu einem beutelartigen Behältnis mit mindestens einer Kammer ausgebildet werden und jede Kammer mit einem weichelastischen Material gefüllt wird, das nach Einlegen in ein Formwerkzeug einer Vulkanisierung unterzogen wird. Hierbei wird zunächst wenigstens eine Außenkammer mit einem ersten Material gefüllt, welches außerhalb des Formwerkzeuges zumindest teilweise ausvulkanisiert wird, worauf wenigstens eine Innenkammer mit einem zweiten Material gefüllt wird und der so vorbereitete Prothesenkörper im Formwerkzeug ausvulkanisiert wird. Bei dem zweiten Material für die Innenkammer handelt es sich um eine mit Leichtfüllstoff versetzte Silikonmasse, die z.B. Mikro-Glaskugeln oder organische Füllstoffe enthält. Als zweites Material kann aber auch ein Polyurethanschaum oder ein Silikonschaum verwendet werden.

Das zweite Material wird in die Innenkammer eingespritzt, d.h. es handelt sich auch bei der zweiten Alternative nicht um einen vorgeformten Schaumstoff; das zweite Material hat also keine Formhaltigkeit. Ferner ist dieses Material mit den Kunststofffolien, die die mit dem ersten Material gefüllten Außenkammern bilden, nicht fest verbunden.

Aus der DE-A-39 42 608 ist ein Verfahren zur Herstellung von Brustprothesen bekannt, die einen Beutel aus zwei an ihren Rändern miteinander verbundenen Polyurethanfolien aufweisen, der mit Silikon gefüllt ist. Zwischen beiden Polyurethanfolien ist eine Schmelzklebefolie eingelegt, die mit einer Ausnehmung versehen ist und die Form eines Ringes hat, wobei die drei so übereinander angeordneten Folien auf den Rand eines Hohlkörperwerkzeuges aufgelegt werden, welche unter Offenlassen eines Zuführkanals geschlossen wird. Anschließend erfolgt die Füllung, indem das Silikonmaterial über den Zuführungskanal zwischen die beiden äußeren Folien eingeführt wird, worauf das Werkzeug erhitzt und das Silikon vernetzt wird, so dass die drei Folien miteinander verbunden werden. Auf der Schmelzklebefolie kann ein mehr oder weniger starrer zusätzlicher Prothesekörper aus Schaumstoff angeordnet werden, der bei der Erwärmung der Schmelzklebefolie mit dieser verbunden wird. Der zusätzliche Prothesenkörper kann aber auch direkt auf der Folie, beispielsweise durch Aufschäumen, erzeugt werden. Der zusätzliche Prothesekörper ist aber nicht fest mit den Polyurethanfolien verbunden. Außerdem wird durch die Verwendung einer Schmelzklebefolie das Verfahren kompliziert.

Der Erfindung lag die Aufgabe zugrunde, eine leichte Brustprothese bereitzustellen, bei der ein Einsatzkörper aus einem Schaumkunststoff in der Prothese fixiert ist und auch nicht in der Lage ist, Wasserdampf aufzunehmen.

Gegenstand der Erfindung ist somit eine Brustprothese gemäß Anspruch 1. Die Merkmale des Oberbegriffs von Anspruch 1 sind aus der DE-A-197 54 144 bekannt.

Durch diese Ausgestaltung wird erreicht, dass der Einsatzkörper innerhalb der Prothese nicht mehr beweglich ist und keinen Wasserdampf aufzunehmen vermag.

Die erfindungsgemäßen Brustprothese ist vorzugsweise dadurch gekennzeichnet, dass der Weichkunststoff des vorderen und des rückwärtigen Teils einen Silikonkunststoff oder eine Polyurethangel darstellt. Ferner kann der Weichkunststoff des rückwärtigen Teils weicher sein als der des vorderen Teils, wodurch er sich besser an den Körper, d.h. das Narbengewebe der operierten Brust, anpasst. Der Weichkunststoff des vorderen Teils hat vorzugsweise eine Härte, ausgedrückt als Penetration, von etwa 160 bis 200 1/10 mm; der Weichkunststoff des rückwärtigen Teils eine Penetration von etwa 200 bis 230 1/10 mm. Die Penetration wird in Anlehnung an DIN 51580 als Eindringtiefe eines Prüfkonus von 65 g in Zehntel-Millimeter definiert. Wünscht man andererseits eine bessere Stabilität, so kann der Weichkunststoff des rückwärtigen Teils härter als der des vorderen Teils sein.

Vorzugsweise sind die thermoplastischen Folien des vorderen und des rückwärtigen Teils Folien auf der Basis von Polyurethan.

Die körperseitige Folie des schalenförmigen rückwärtigen Teils braucht nicht flächig auf der Haut zu liegen, um das Narbengewebe nicht zu irritieren.

Die schalenförmigen Teile haben im allgemeinen eine Wandstärke, die sich bis zum Rand hin vermindert, beispielsweise von 10 bis 30 mm bis auf 0 mm.

Der Schaumkunststoff des Einsatzkörpers kann einen Schaumstoff auf der Basis von Weich-Polyurethan darstellen. Auch ein Silikonschaum oder Epoxidschaum ist geeignet. Die Stauchhärte des Schaumkunststoffs beträgt etwa 1,5 bis 7,0 kPa (40%). Der Schaumkunststoff hat vorzugsweise ein Bruttoraumgewicht von etwa 18 bis 30, insbesondere von 25 kg/m³.

Der Einsatzkörper ist durch Vertiefungen (Kanäle) unterteilt, die durch Stege miteinander verbunden sind, wobei die Vertiefungen auf der Seite des vorderen Teils offen und auf der Seite des rückwärtigen Teils geschlossen sind. Durch die Vertiefungen im Einsatzkörper wird ein Druckausgleich gewährleistet, und die Elastizität wird erhöht. Die Starrheit wird herabgesetzt, und es wird ein natürliches Schwingungsverhalten erhalten. Die Stege sind auf der dem Körper anliegenden Seite miteinander verbunden.

Vorzugsweise verlaufen die Vertiefungen im wesentlichen vertikal.

Die Vertiefungen können aus dem Einsatzkörper ausgefräst sein. Sie können aber auch beim Ausschäumen des Einsatzkörpers erzeugt werden.

Vorzugsweise enthält der Einsatzkörper mindestens vier Vertiefungen, insbesondere vier bis sechs Vertiefungen, wobei jede Vertiefung etwa 5 bis 15 mm breit ist. Die Vertiefungen stehen im allgemeinen senkrecht zu der Rückseite, sie können jedoch auch aufgefächert, sein.

Gegenstand der Erfindung ist auch ein Verfahren zur Herstellung der erfindungsgemäßen Brustprothesen, welches durch folgende Schritte gekennzeichnet ist.
a.1) Einlegen einer ersten thermoplastischen Folie in eine erwärmte Matrize;
a.2) Einfüllen eines aushärtbaren Weichkunststoffs in einer zur Bildung des schalenförmigen vorderen Teils ausreichenden Menge;
a.3) Auflegen einer zweiten thermoplastischen Folie auf den Weichkunststoff;
a.4) Einführen einer Patrize in die Matrize, wobei zwischen Patrize und Matrize ein Volumen freigelassen wird, das dem Volumen des vorderen Teils entspricht;
a.5) Verbinden der Ränder der ersten und der zweiten thermoplastischen Folie;
a.6) gegebenenfalls Aushärten des schalenförmigen vorderen Teils;
b.) Erzeugen des rückwärtigen schalenförmigen Teils in einer getrennten Form, Verschweißung der Ränder und gegebenenfalls Aushärten des schalenförmigen rückwärtigen Teils, analog zur Herstellung des vorderen schalenförmigen Teils;
c.) Einlegen des vorgeformten, schaumförmigen Einsatzkörpers und Verkleben von dessen Vorderseite mit der zweiten thermoplastischen Folie des vorderen Teils;
d.) Auflegen des rückwärtigen, schalenförmigen Teils auf die Rückseite des Einsatzkörpers und Verkleben des Einsatzkörpers (5) mit dem rückwärtigen schalenförmigen Teil (6) ;
e.) Verschweißen des vorderen (1) mit dem rückwärtigen (6) schalenförmigen Teil;
f.) Aushärten des Klebstoffs und, falls dies nicht bereits in Stufe (a.5) bzw. (b.) geschehen ist, des schalenförmigen vorderen und rückwärtigen Teils;
g.) Entnahme der fertigen Brustprothese aus der Form.

Vorzugsweise verwendet man als Kleber einen Silikonkleber.

Der Einsatzkörper wird vorzugsweise dadurch hergestellt, dass man ihn aus einem größeren Schaumstoffkörper ausschneidet und gegebenenfalls Vertiefungen einschneidet; oder den Einsatzkörper gegebenenfalls mit Vertiefungen durch Aushärten und Ausschäumen in einer Form herstellt.

Die Erfindung ist anhand der beigefügten Zeichnung erläutert.
Es zeigen
- Fig. 1: einen senkrechten Schnitt durch die Prothese gemäß der Erfindung;
- Fig. 2: einen Horizontalschnitt durch eine bevorzugte Ausführungsform der vorliegenden Erfindung;
- Fig. 3: einen horizontalen Schnitt durch den Einsatzkörper.

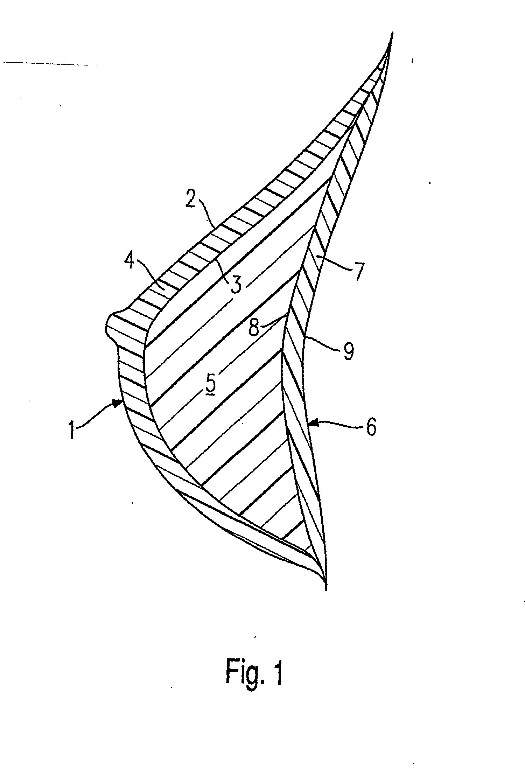

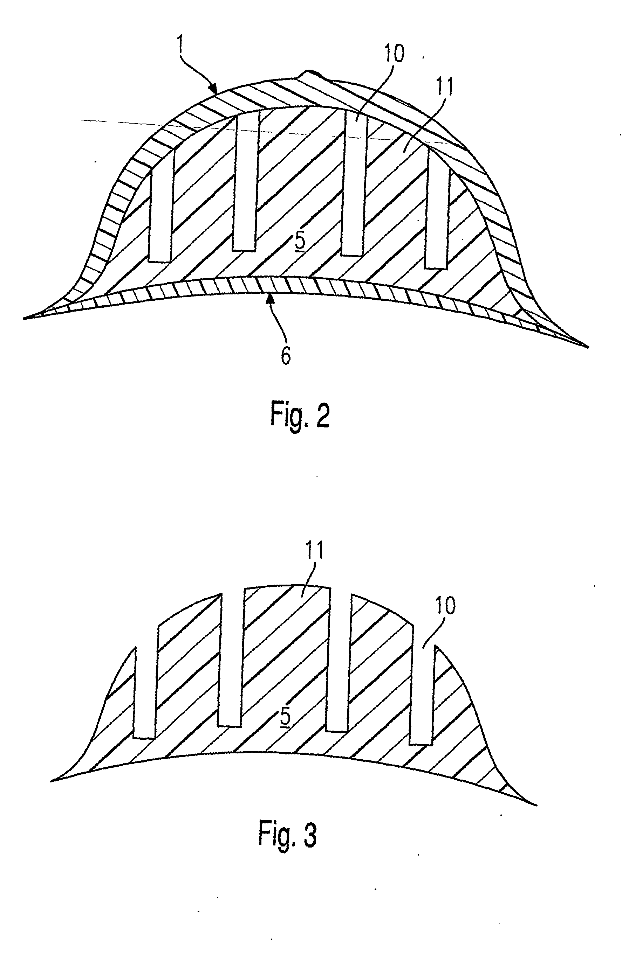

Die in den Figuren 1 und 2 dargestellten Brustprothesen enthalten ein schalenförmiges vorderes Teil 1, mit einem zwischen den Polyurethanfolien 2 und 3 eingeschlossenen und ausgehärteten Weichkunststoff 4, z.B. einem Silikonkunststoff mit einer Penetration von etwa 160 bis 200 1/10 mm. Etwa in der Mitte des schalenförmigen Teils 1 ist eine Erhöhung vorgesehen, die eine Brustwarze andeuten soll. Das schalenförmige Teil 1 ist an dieser Stelle am stärksten, und die Wandstärke nimmt zum Rande hin gegen 0 ab. Die Folien 2 und 3 sind am Rand durchgehend miteinander verschweißt.

Mit der inneren Folie 3 ist die äußere Oberfläche des Einsatzkörpers 5 so verklebt, dass zwischen der Folie 3 und dem Einsatzkörper 5 keine Hohlräume entstehen. Der Einsatzkörper folgt den Konturen des schalenförmigen vorderen Teils 1. Der Einsatzkörper 5 kann aus einem größeren Schaumstoffteil herausgeschnitten oder anderweitig geformt werden. Er kann aber auch in einer entsprechenden Form aus den Ausgangskomponenten unter Zusatz eines Schaumbildners aufgeschäumt und ausgehärtet werden. Die Form ist so ausgebildet, dass der erhaltene Einsatzkörper 5 wiederum den Konturen des vorderen schalenförmigen Teils 1 folgt.

Mit der rückwärtigen Oberfläche des Einsatzkörpers 5 ist das rückwärtige schalenförmige Teil 6 verklebt. Der Weichkunststoff 7 des Teils 6 ist ähnlich wie der Weichkunststoff 4 des vorderen schalenförmigen Teils 1 zwischen den Folien 8 und 9 eingeschlossen. Die Dicke des Teils 6 vermindert sich wie die des Teils 1 zum Rande hin, so dass die Folien 8 und 9 dort unmittelbar miteinander verschweißt sind. Ferner sind die Folien 2 und 3 am Rand durchgehend mit den Folien 8 und 9 verschweißt.

In Fig. 1 sind keine Vertiefungen 10 dargestellt. Dies beruht darauf, dass diese, wie in der Fig. 2 dargestellt, in der horizontalen Schnittebene liegen. Die Vertiefungen brauchen aber nicht unbedingt vorhanden zu sein. In Fig. 2 sind die mit 10 bezeichneten Vertiefungen (insgesamt vier) rechteckig ausgestaltet und durch die Stege 11 voneinander getrennt. Die Vertiefungen 10 sind nicht ganz bis zum rückwärtigen Teil 6 durchgeführt, sondern enden etwa 5 bis 10 mm über diesem. Durch die Anordnung der Vertiefungen erhält die Brustprothese eine größere Elastizität und ein ideales Schwingverhalten.

Fig. 3 zeigt den Einsatzkörper vor dem Einbau in die Brustprothese, wobei für gleiche Elemente die in Fig. 1 und 2 angegebenen Bezugszahlen verwendet wurden.

Die erfindungsgemäße Brustprothese kann beispielsweise unter Verwendung einer beheizten Form, wie sie in der DE-C-39 38 328 beschrieben ist, hergestellt werden, die für den vorliegenden Fall etwas modifiziert ist.

## Patentansprüche

1. Brustprothese, enthaltend ein der Brustform nachgebildetes schalenförmiges Vorderteil (1) mit einem zwischen zwei thermoplastischen Folien (2, 3) eingeschlossenen Weichkunststoff (4), ein dem Körper zugewandtes, rückwärtiges Teil (6), mit einem zwischen zwei thermoplastischen Folien (8, 9) eingeschlossenen Weichkunststoff (7), wobei das vordere (1) und das rückwärtige (6) Teil am Außenrand durchgehend miteinander verschweißt sind; und eine zwischen dem vorderen (1) und dem rückwärtigen (6) Teil eingeschlossenen und mit diesen verbundenen Einsatzkörper (5) aus einem Schaumkunststoff, der mit den inneren Folien (3, 8) des vorderen (1) und des rückwärtigen (6) Teils verklebt ist, **dadurch gekennzeichnet, dass** der vorgefertigte Einsatzkörper (5) durch Vertiefungen (Kanäle) (10) unterteilt ist, die durch Stege (11) miteinander verbunden sind.

2. Brustprothese nach Anspruch 1, **dadurch gekennzeichnet, dass** der Weichkunststoff (4) bzw. (7) des vorderen (1) und des rückwärtigen (6) Teils einen Silikonkunststoff oder ein Polyurethangel darstellt.

3. Brustprothese nach Anspruch 1 oder 2, **dadurch gekennzeichnet, dass** der Weichkunststoff (7) des rückwärtigen Teils (6) weicher ist als der Weichkunststoff (4) des vorderen Teils (1).

4. Brustprothese nach einem der Ansprüche 1 bis 3, **dadurch gekennzeichnet, dass** die thermoplastischen Folien (2, 3; 8, 9) des vorderen (1) und des rückwärtigen (6) Teils Folien auf der Basis von Polyurethan darstellen.

5. Brustprothese nach einem der Ansprüche 1 bis 4, **dadurch gekennzeichnet, dass** der Schaumkunststoff des Einsatzkörpers (5) einen Schaumstoff auf der Basis von Weich-Polyurethan darstellt.

6. Brustprothese nach einem der vorstehenden Ansprüche, **dadurch gekennzeichnet, dass** die Vertiefungen (10) im wesentlichen vertikal verlaufen.

7. Brustprothese nach einem der vorstehenden Ansprüche, **dadurch gekennzeichnet, dass** die Vertiefungen (10) aus dem Einsatzkörper (5) ausgefräst sind.

8. Brustprothese nach einem der vorstehenden Ansprüche, **dadurch gekennzeichnet, dass** die Vertiefung (10) beim Aufschäumen des Einsatzkörpers (5) erzeugt werden.

9. Verfahren zur Herstellung der Brustprothese nach einem der Ansprüche 1 bis 8, **gekennzeichnet durch** folgende Schritte:
a.1) Einlegen einer ersten thermoplastischen Folie (2) in eine erwärmte Matrize;
a.2) Einfüllen eines aushärtbaren Weichkunststoffs (4) in einer zur Bildung des schalenförmigen vorderen Teils (1) ausreichenden Menge;
a.3) Auflegen einer zweiten thermoplastischen Folie (3) auf den Weichkunststoff (4);
a.4) Einführen einer Patrize in die Matrize, wobei zwischen Patrize und Matrize ein Volumen freigelassen wird, das dem Volumen des vorderen Teils (1) entspricht;
a.5) Verbinden (Verschweißen) der Ränder der ersten (2) und der zweiten (3) thermoplastischen Folie;
a.6) gegebenenfalls Aushärten des schalenförmigen vorderen Teils (1);
b.) Erzeugen des rückwärtigen schalenförmigen Teils (6) in einer getrennten Form, Verschweißung der Ränder und Aushärtung des schalenförmigen rückwärtigen Teils (6), analog zur Herstellung des vorderen schalenförmigen Teils (1);
c.) Einlegen des vorgeformten schaumförmigen Einsatzkörpers (5) und Verkleben von dessen Vorderseite mit der zweiten thermoplastischen Folie (3) des vorderen Teils (1);
d.) Auflegen des rückwärtigen schalenförmigen Teils (6) auf die Rückseite des Einsatzkörpers (5) und Verkleben des Einsatzkörpers (5) mit dem rückwärtigen schalenförmigen Teil (6);
e.) Verschweißen des vorderen (1) mit dem rückwärtigen (6) schalenförmigen Teil;
f.) Aushärten des Klebstoffs und, falls dies nicht in Stufe (a.5) bzw. (b.) geschehen ist, des schalenförmigen vorderen (1) und rückwärtigen (6) Teils;
g.) Entnahme der fertigen Brustprothese aus der Form.

10. Verfahren nach Anspruch 9, **dadurch gekennzeichnet, dass** man als Kleber einen Polyurethan- oder Silikonkleber verwendet.

11. Verfahren nach Anspruch 9 oder 10, **dadurch gekennzeichnet, dass** man den Einsatzkörper (5) aus einem größeren Schaumstoffkörper ausschneidet und gegebenenfalls Vertiefungen (10) einschneidet; oder den Einsatzkörper (5) gegebenenfalls mit Vertiefungen (10) durch Aushärten und Ausschäumen in einer Form herstellt.

## Claims

1. A breast prosthesis, comprising a cup-shaped front part (1) imitating a breast shape, with a soft plastics material (4) enclosed between two thermoplastic sheets (2,3), a rear part (6) facing the body, with a soft plastics material (7) enclosed between two thermoplastic sheets (8,9), wherein the front part (1) and the rear part (6) are welded together continuously at the outer edge; and an insert member (5) which is enclosed between the front part (1) and the rear part (6) and is connected thereto, and which consists of a foamed plastic which is bonded to the inner sheets (3,8) of the front part (1) and rear part (6), **characterised in that** the prefabricated insert member (5) is divided up by recesses (channels) (10) which are connected to one another by webs.

2. A breast prosthesis according to Claim 1, **characterised in that** the soft plastics material (4) and (7) of the front part (1) and the rear part (6) respectively represents a silicone plastic or a polyurethane gel.

3. A breast prosthesis according to Claim 1 or 2, **characterised in that** the soft plastics material (7) of the rear part (6) is softer than the soft plastics material (4) of the front part (1) .

4. A breast prosthesis according to any one of Claims 1 to 3, **characterised in that** the thermoplastic sheets (2,3;8,9) of the front part (1) and of the rear part (6) represent polyurethane-based sheets.

5. A breast prosthesis according to any one of Claims 1 to 4, **characterised in that** the foamed plastic of the insert member (5) represents a soft polyurethane-based foamed material.

6. A breast prosthesis according to any one of the preceding Claims, **characterised in that** the recesses (10) extend substantially vertically.

7. A breast prosthesis according to any one of the preceding Claims, **characterised in that** the recesses (10) are milled from the insert member (5).

8. A breast prosthesis according to any one of the preceding Claims, **characterised in that** the recesses (10) are formed during the foaming of the insert member (5).

9. A method of producing the breast prosthesis according to any one of Claims 1 to 8, **characterised by** the following steps:
a.1) inserting a first thermoplastic sheet (2) in a heated female die;
a.2) charging a curable soft plastics material (4) in a quantity sufficient to form the cup-shaped front part (1);
a.3) applying a second thermoplastic sheet (3) on the soft plastics material (4);
a.4) introducing a male die into the female die, leaving free between the male die and the female die a volume which corresponds to the volume of the front part (1);
a.5) joining (welding) the edges of the first (2) and the second (3) thermoplastic sheet;
a.6) optionally curing the cup-shaped front part (1);
b.) creating a rear cup-shaped part (6) in a separate mould, welding the edges and curing the cup-shaped rear part (6), in an analogous manner to the production of the cup-shaped front part (1)
c.) inserting the preformed foam-like insert member (5) and bonding its front side to the second thermoplastic sheet (3) of the front part (1);
d.) applying the rear cup-shaped part (6) on to the rear side of the insert member (5) and bonding the insert member (5) to the rear cup-shaped part (6);
e.) welding the front (1) to the rear (6) cup-shaped part;
f.) curing the adhesive and, if this has not taken place in step (a.5) or (b.), the cup-shaped front (1) and rear (6) part;
g.) removing the finished breast prosthesis from the mould.

10. A method according to Claim 9, **characterised in that** a polyurethane or silicone adhesive is used as the adhesive.

11. A method according to Claim 9 or 10, **characterised in that** the insert member (5) is cut out from a larger foamed material member and optionally recesses (10) are cut therein; or the insert member (5) is optionally produced with recesses (10) by curing and expanding in a mould.

## Revendications

1. Prothèse mammaire, contenant une partie avant (1) en forme de coque reproduisant la forme de poitrine avec un plastique souple (4) inclus entre deux films (2, 3) thermoplastiques, une partie (6) arrière, associée au corps, avec un plastique souple (7) inclus entre deux films (8, 9) thermoplastiques, la partie avant (1) et la partie arrière (6) étant soudées l'une avec l'autre de façon continue sur le bord extérieur, et un corps d'insert (5) enfermé entre la partie avant (1) et la partie arrière (6) et relié à celle-ci, à base d'un plastique cellulaire, qui est collé avec les films (3, 8) intérieurs de la partie avant (1) et de la partie arrière (6), **caractérisée en ce que** le corps d'insert (5) préfabriqué est subdivisé par des cavités (canaux) (10), qui sont reliées entre elles par des nervures (11).

2. Prothèse mammaire selon la revendication 1, **caractérisée en ce que** le plastique souple (4) ou (7) de la partie avant (1) et de la partie arrière (6) représente un plastique silicone ou un gel de polyuréthane.

3. Prothèse mammaire selon la revendication 1 ou 2, **caractérisée en ce que** le plastique souple (7) de la partie (6) arrière est plus souple que le plastique souple (4) de la partie avant (1).

4. Prothèse mammaire selon l'une quelconque des revendications 1 à 3, **caractérisée en ce que** les films (2, 3 ; 8, 9) thermoplastiques de la partie avant (1) et de la partie arrière (6) représentent des films à base de polyuréthane.

5. Prothèse mammaire selon l'une quelconque des revendications 1 à 4, **caractérisée en ce que** le plastique cellulaire du corps d'insert (5) représente une mousse à base de polyuréthane souple.

6. Prothèse mammaire selon l'une quelconque des revendications précédentes, **caractérisée en ce que** les cavités (10) sont agencées sensiblement verticalement.

7. Prothèse mammaire selon l'une quelconque des revendications précédentes, **caractérisée en ce que** les cavités (10) sont fraisées à partir du corps d'insert (5).

8. Prothèse mammaire selon l'une quelconque des revendications précédentes, **caractérisée en ce que** les cavités (10) sont générées lors du moussage du corps d'insert (5).

9. Procédé pour fabriquer la prothèse mammaire selon l'une quelconque des revendications 1 à 8, **caractérisé par** les étapes suivantes :
a.1) insertion d'un premier film (2) thermoplastique dans une matrice chauffée ;
a.2) versement d'un plastique souple (4) durcissable en une quantité suffisante pour former la partie (1) avant en forme de coque ;
a.3) pose d'un second film (3) thermoplastique sur le plastique souple (4) ;
a.4) introduction d'une patrice dans la matrice, un volume, qui correspond au volume de la partie (1) avant, étant libéré entre la patrice et la matrice ;
a.5) assemblage (soudage) des bords du premier (2) et du second (3) film thermoplastique ;
a.6) éventuellement durcissement de la partie (1) avant en forme de coque ;
b.) génération de la partie (6) arrière en forme de coque dans une forme séparée, soudage des bords et durcissement de la partie (6) arrière en forme de coque, par analogie avec la fabrication de la partie (1) avant en forme de coque ;
c.) insertion du corps d'insert (5) en forme de mousse préformé et collage de sa partie avant avec le second film (3) thermoplastique de la partie (1) avant ;
d.) pose de la partie (6) arrière en forme de coque sur la face arrière du corps d'insert (5) et collage du corps d'insert (5) avec la partie (6) arrière en forme de coque ;
e.) soudage de la partie avant (1) à la partie arrière (6) en forme de coque ;
f.) durcissement de la coque et, si cette opération n'intervient pas au niveau (a.5) ou (b.) de la partie avant (1) et arrière (6) en forme de coque ;
g.) prélèvement de la prothèse mammaire terminée du moule.

10. Procédé selon la revendication 9, **caractérisé en ce qu'**on utilise comme colle une colle à base de polyuréthane ou de silicone.

11. Procédé selon la revendication 9 ou 10, **caractérisé en ce qu'**on découpe le corps d'insert (5) dans un corps de mousse assez grand et incise éventuellement des cavités (10) ou qu'on fabrique le corps d'insert (5) éventuellement avec des cavités (10) par durcissement et moussage dans un moule.
